# DEMANDE DE BREVET EUROPEEN

(11) **EP 1 929 981 A1**
(43) Date de publication de la demande: **11.06.2008**
(21) Numéro de dépôt: 07122499.2
(22) Date de dépôt: 06.12.2007
(51) Int. Cl.: A61F 7/10

(54) **Dispositif de refroidissement**

(30) Priorité: 07.12.2006 FR 0655362
(71) Demandeur: L'Oréal, 75008 Paris (FR)
(72) Inventeur: Sanchez, Marcel, 93600, Aulnay sous Bois (FR)
(74) Mandataire: Julio, Charlotte

(57) **Abrégé**

La présente invention concerne un dispositif de refroidissement comprenant :
- des moyens de refroidissement (1) comportant un distributeur de produit réfrigérant comprenant un récipient aérosol (10) contenant ledit produit réfrigérant et une valve de distribution (2) associée à une coupelle de fixation à sertir sur le récipient aérosol, ledit récipient aérosol étant au moins en partie logé à l'intérieur d'une coque d'habillage (5),
- un conduit de distribution (40) engagé sur ladite valve de distribution (2),
- un couvercle (6) surmontant ledit conduit de distribution (40),

caractérisé en ce que ledit couvercle (6) comporte une face interne (602) imperméable au produit réfrigérant et en ce qu'au moins une partie du couvercle, notamment une face externe du couvercle (601), est réalisée en un matériau présentant une conductivité thermique supérieure ou égale à 1 W·m⁻¹·K⁻¹. La présente invention concerne également un procédé d'application d'un produit cosmétique sur une surface corporelle.

## Description

La présente invention concerne un dispositif de refroidissement.

Une application privilégiée, mais non exclusive, de la présente invention vise à refroidir une surface corporelle dans le but d'améliorer localement la pénétration un produit cosmétique. Par « produit cosmétique », on entend un produit tel que défini dans la Directive 93/35/CEE du Conseil du 14 juin 1993.

Il est avantageux dans certains cas de réfrigérer une surface corporelle afin de conférer à l'utilisateur une sensation de fraîcheur et de bien être. De même, il est parfois souhaitable d'appliquer un produit cosmétique à basse température afin d'accroître l'effet dudit produit.

Ainsi, afin de refroidir ledit produit cosmétique, l'utilisateur peut maintenir le produit au réfrigérateur avant de l'appliquer. Toutefois, un inconvénient lié à cette méthode est qu'en périodes de fortes chaleurs, une fois sorti du réfrigérateur, le produit cosmétique se réchauffe rapidement. L'utilisateur ne peut ainsi plus bénéficier de l'effet rafraîchissant recherché.

Des dispositifs destinés à refroidir une surface corporelle ont donc été développés dans l'art antérieur.

Le document EP 0 608 954 divulgue un exemple de dispositif de l'art antérieur apte à refroidir une surface cutanée. Ce dispositif comporte un récipient pressurisé contenant un produit réfrigérant. Ce récipient est muni d'une valve de distribution adaptée à sélectivement distribuer ledit produit réfrigérant. Cette valve est pourvue d'un orifice apte à communiquer avec un conduit de distribution. Ce conduit de distribution débouche sur un applicateur poreux destiné à appliquer ledit liquide réfrigérant sur une surface à traiter.

Toutefois, un inconvénient lié à un tel dispositif est que la surface de l'applicateur en contact avec la peau est particulièrement restreinte de sorte que le traitement est très localisé. De plus, le produit réfrigérant distribué étant compris entre -20°C et 0°C, un tel froid ne peut être maintenu longtemps au contact de la peau au risque autrement de brûler la peau. Une application de froid prolongée au moyen d'un tel dispositif ne peut donc être envisagée.

La présente invention a donc pour but de surmonter les inconvénients susmentionnés.

La présente invention a également pour but de réaliser un dispositif de refroidissement apte à maintenir longtemps un effet froid sur la surface corporelle à traiter.

La présente invention a encore pour but de réaliser un dispositif rechargeable, facile à prendre en main.

La présente invention a aussi pour but de réaliser un dispositif facile à fabriquer, à mettre en oeuvre et présentant un coût de revient peu élevé.

Pour ce faire, la présente invention a donc pour objet un dispositif de refroidissement comprenant :
- des moyens de refroidissement comportant un distributeur de produit réfrigérant comprenant un récipient aérosol contenant ledit produit réfrigérant et une valve de distribution associée à une coupelle de fixation à sertir sur le récipient aérosol, ledit récipient aérosol étant au moins en partie logé à l'intérieur d'une coque d'habillage,
- un conduit de distribution engagé sur ladite valve de distribution,
- un couvercle surmontant ledit conduit de distribution,
dans lequel ledit couvercle comporte une face interne imperméable au produit réfrigérant et en ce qu'au moins une partie du couvercle, notamment une face externe du couvercle, est réalisée en un matériau présentant une conductivité thermique supérieure ou égale à 1 W·m⁻¹·K⁻¹.

Il est à noter que ce couvercle peut être réalisé monobloc avec la coque d'habillage ou selon un mode de réalisation avantageux peut être rapporté sur la coque d'habillage. Autrement dit, dans ce deuxième cas, le couvercle vient en prise fixante avec la coque d'habillage.

Ladite conductivité thermique peut être comprise entre 10 et 430 W·m⁻¹·K⁻¹, et plus préférablement entre 20 et 240 W·m⁻¹·K⁻¹.

Ledit matériau présentant une conductivité thermique supérieure ou égale à 1 W·m⁻¹·K⁻¹ peut être un matériau métallique et notamment du titane, de l'acier, du platine ou de l'aluminium.

Ledit récipient peut comporter une première extrémité pourvue de ladite valve de distribution et une deuxième extrémité fermée, lesdites première et deuxième extrémités étant reliées par une paroi latérale, ladite coque d'habillage enveloppant au moins partiellement ladite paroi latérale.

Ledit couvercle peut être amovible.

En position de montage du couvercle, ledit couvercle est apte à venir en butée contre ladite coque d'habillage.

En position de montage du couvercle, lesdites faces interne et externe peuvent être orientées de façon invariable relativement audit conduit de distribution.

Le dispositif peut comprendre des moyens d'immobilisation dudit couvercle sur ledit conduit de distribution et/ ou sur ladite coque d'habillage.

Ledit couvercle peut être fixé sur ledit conduit de distribution par emmanchement en force, par vissage ou par encliquetage.

Ladite coque d'habillage et ledit conduit de distribution peuvent être réalisées monobloc.

Le dispositif peut comprendre des moyens de réglage du volume délimité entre ledit conduit de distribution et ledit couvercle.

Ladite face externe du couvercle présente une surface convexe.

La présente invention a également pour objet un procédé d'application d'un produit cosmétique sur une surface corporelle comprenant une première étape d'appliquer ledit produit cosmétique sur la surface corporelle suivie d'une deuxième étape de masser ladite surface corporelle recouverte de produit cosmétique à l'aide d'un dispositif selon l'une quelconque des revendications précédentes.

L'invention pourra être mieux comprise à la lecture de la description détaillée suivante, faite en référence aux dessins accompagnants illustrant un mode de réalisation non limitatif de celle-ci, et sur lesquels :
- la figure 1 est une vue schématique en perspective d'un mode de réalisation d'un dispositif selon l'invention,
- la figure 2 est une vue schématique en section longitudinale selon le plan de coupe A-A du dispositif de la figure 1,
- la figure 3 est vue schématique en perspective d'un autre mode de réalisation d'un dispositif selon l'invention,
- la figure 4 est une vue schématique en section longitudinale selon le plan de coupe B-B du dispositif de la figure 3,
- la figure 5 est vue schématique en perspective d'un autre mode de réalisation d'un dispositif selon l'invention,
- la figure 6 est une vue schématique en section longitudinale selon le plan de coupe C-C du dispositif de la figure 5,
- la figure 7 est vue schématique en perspective d'un autre mode de réalisation d'un dispositif selon l'invention,
- la figure 8 est une vue schématique en section longitudinale selon le plan de coupe D-D du dispositif de la figure 7,
- la figure 9 est un graphique représentant l'aptitude de différents matériaux à se réchauffer en fonction du temps.

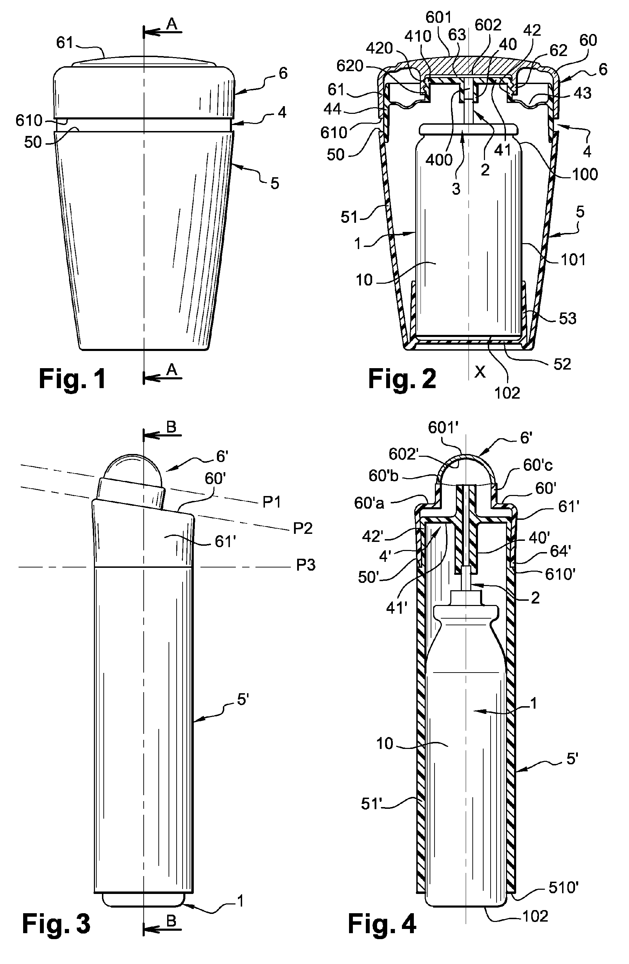

En références aux figures 1 et 2, le dispositif selon l'invention comporte des moyens de refroidissement 1.

Ces moyens de refroidissement 1 comportent un distributeur de produit réfrigérant comprenant un récipient aérosol 10 contenant ledit produit réfrigérant. Le produit réfrigérant est par exemple un gaz à l'état liquéfié lorsqu'il est sous pression dans le récipient et dont la température chute à l'état vaporisé lorsqu'il se détend. Ce produit réfrigérant peut par exemple être un hydrocarbure tel que le butane-3, 2, le diméthyléther ou le propane, ou encore un fréon tel que le HFC 134a.

Le récipient aérosol comporte une première extrémité ouverte 100 et une deuxième extrémité fermée 102, lesdites première et deuxième extrémités étant reliées par une paroi latérale 101. Ce récipient peut présenter une forme générale de cylindre de révolution.

La première extrémité 100 est équipée d'une valve de distribution 2. Cette valve de distribution 2 est adaptée à sélectivement distribuer le produit réfrigérant contenu dans ledit récipient aérosol. La valve de distribution 2 peut être associée au récipient aérosol de façon conventionnelle par une coupelle de fixation 3 à sertir sur ladite première extrémité ouverte 100 du récipient aérosol. Cette valve de distribution peut être sertie extérieurement ou intérieurement sur ladite première extrémité ouverte du récipient. Un sertissage interne est également connu sous le terme de dudgeonnage.

La valve de distribution 2 peut être une valve à actionnement par enfoncement et/ou par basculement, à libération dosée ou continue de produit réfrigérant. Une telle valve comprend généralement une tige de valve actionnable par déplacement relativement au récipient. Cette tige de valve peut être déplacée relativement à un corps de valve. Ce corps de valve peut éventuellement définir une chambre de dosage du produit réfrigérant à distribuer. Un tel corps de valve peut être retenu sur la première extrémité ouverte du récipient au moyen de ladite coupelle de fixation à sertir. Une valve à enfoncement comporte en particulier une tige de valve dotée d'un mouvement de va-et-vient relativement audit corps de valve et plus généralement relativement au récipient. Cette valve, et en particulier la tige de valve, définit généralement intérieurement un canal d'expulsion dudit produit réfrigérant apte à établir une communication sélective avec le corps de valve et plus généralement avec ledit contenu du récipient. Ainsi, lorsque la tige de valve est déplacée par un utilisateur, ledit canal d'expulsion peut communiquer avec l'intérieur dudit récipient aérosol et ledit produit réfrigérant peut ainsi être distribué.

Dans le présent exemple de réalisation, la valve de distribution 2 comporte une tige de valve à actionnement par enfoncement. Cette tige de valve peut être déplacée axialement par rapport au récipient pressurisé 1 entre une position de repos et une position de distribution. Pour ce faire, cette tige de valve est généralement emmanchée dans un organe de d'actionnement 4. Une fois emmanchée, la tige de valve est solidaire en déplacement dudit organe d'actionnement 4. L'actionnement de la valve est alors déclenchée par un déplacement relatif entre ledit organe d'actionnement 4 et ledit récipient aérosol 10.

Le récipient aérosol est logé dans une coque d'habillage 5. Cette coque d'habillage enveloppe au moins en partie la paroi latérale101 du récipient.

Comme représenté sur les figures 1 et 2, la coque d'habillage 5 peut comporter un bord libre 50 définissant une ouverture, par exemple circulaire, et un fond 52. Ce bord libre et ce fond sont reliés par une paroi cylindrique 51. Ladite coque présente une forme générale de godet.

Le récipient 1 peut être introduit à travers l'ouverture de la coque d'habillage. Dans cet exemple, la coque s'étend sur toute la hauteur du récipient. Le bord libre 50 de la coque vient en affleurement de la première extrémité ouverte 100 de sorte que ladite coque entoure totalement ledit récipient pressurisé.

Cette coque d'habillage définit une surface de préhension. Cette coque est de préférence au moins partiellement réalisée en un matériau présentant une conductivité thermique inférieure à 1 Wm⁻¹K⁻¹. Cette coque peut par exemple comporter un matériau plastique comme le polyéthylène, le polyamide ou le poly oxy méthylène ou un matériau alvéolaire telle qu'une mousse. De préférence, la coque est opaque afin de masquer complètement ledit récipient. Toutefois, une échancrure pourrait éventuellement être ménagée dans ladite coque de sorte que le récipient serait en partie visible de l'extérieur. Une telle coque présente ainsi d'une part une fonction esthétique et d'autre part facilite la prise en main du dispositif.

La coque d'habillage 5 peut en outre comporter des moyens de retenue 53 se dressant à partir du fond de la coque 52. Ces moyens de retenue comprennent une couronne dans laquelle peut être insérée, de préférence en prise serrante, une partie du récipient aérosol. La partie logée dans cette couronne peut par exemple s'étendre de la deuxième extrémité fermée 102 jusqu'à un quart du corps de récipient. De tels moyens de retenue limitent alors les déplacements latéraux, et de préférence axiaux, du récipient pressurisé relativement à ladite coque d'habillage 5.

L'organe d'actionnement 4 comprend un conduit de distribution 40, un plateau 41, une jupe périphérique 42, des moyens résilients 43 et une bague de montage 44.

Le conduit de distribution 40 est emmanché sur la tige de valve afin de conduire le produit distribué par la valve 2. Le conduit débouche au travers du plateau 41 par un orifice 400. Ce conduit s'étend selon un axe longitudinal X, qui dans cet exemple est confondu avec un axe de révolution du récipient.

Le plateau 41 s'étend radialement à partir dudit orifice 400. Le plateau 41 comporte un bord externe 410 circulaire.

La jupe périphérique 42 se dresse quant à elle à partir dudit bord externe 410. Cette jupe périphérique comprend une surface externe pourvue de moyens de fixation 420 aptes à coopérer avec un couvercle 6 comme il sera expliqué dans la suite de cette description. Ces moyens de fixation comprennent avantageusement des filets 420.

Les moyens résilients 43 relient la jupe périphérique 42 avec la bague de montage 44. Ces moyens résilients 43 peuvent comprendre une pluralité de pattes flexibles, par exemple au nombre de six, s'étendant, par exemple à intervalles réguliers, entre ladite jupe périphérique 42 et ladite bague de montage 44. Autrement, ces moyens résilients peuvent comprendre un anneau élastique disposé entre ladite jupe périphérique 42 et ladite bague de montage 44.

La bague de montage 44 est engagée en prise serrante dans l'ouverture de la coque d'habillage 5. Cette bague de montage est ainsi montée fixement dans ladite coque d'habillage. Toutefois, les moyens résilients 43 permettent au conduit 40, au plateau 41 et à la jupe périphérique 42 d'être déplacés axialement relativement à ladite bague de montage 44 afin de permettre l'actionnement de la valve de distribution 2.

Le couvercle 6 peut comprendre une partie de recouvrement 60 et une jupe latérale 62.

La partie de recouvrement 60 est placée en vis-à-vis de l'orifice 400 de l'organe d'actionnement 4 de manière à être mis directement au contact du produit distribué. Cette partie de recouvrement comporte une face externe 601 et une face interne 602.

Selon l'invention, la face interne 602 est imperméable au produit réfrigérant. Autrement dit, cette face interne est apte à empêcher le produit réfrigérant sortant du conduit de distribution 40 de traverser le couvercle et de venir directement au contact de la peau. En outre, la couvercle est au moins en partie réalisé en un matériau présentant une conductivité thermique supérieure ou égale à 1 W·m⁻¹·K⁻¹, de préférence comprise entre 10 et 430 W·m⁻¹·K⁻¹, et plus préférablement entre 20 et 240 W·m⁻¹·K⁻¹.

Le couvercle peut donc être entièrement réalisé en un tel matériau. Autrement, seule une partie du couvercle, notamment la partie de recouvrement 60, peut être réalisée en un tel matériau. Dans ce cas, ce matériau peut s'étendre de la face interne à la face externe de la partie de recouvrement de sorte que le froid est aisément conduit à travers cette partie de recouvrement. Une telle conductivité permet alors au produit réfrigérant de refroidir efficacement la face externe de la partie de recouvrement 60 et garantit une bonne répartition du froid dans la partie de recouvrement.

Autrement, la face interne peut être réalisée en un matériau présentant une conductivité thermique inférieure à 1 W·m⁻¹·K⁻¹ et la face externe peut être réalisée en un matériau présentant une conductivité thermique supérieure à 1 W·m⁻¹·K⁻¹ ou inversement.

Le matériau présentant une conductivité thermique supérieure à 1 W·m⁻¹·K⁻¹ peut également être prévu sous forme d'un ou plusieurs insert(s) logés dans la partie de recouvrement. De préférence, cet insert s'étend transversalement à l'axe longitudinal X du conduit de distribution 40. Cet insert peut être noyé dans un matériau présentant une conductivité thermique inférieure à 1 W·m⁻¹·K⁻¹. Un tel insert peut alors être situé à distance de la face externe et de la face interne de la partie de recouvrement. Les face externe et interne de la partie de recouvrement peuvent donc présenter une conductivité thermique inférieure à 1 W·m⁻¹·K⁻¹.

Le matériau présentant une conductivité thermique supérieure ou égale à 1 W·m⁻¹·K⁻¹ peut par exemple être du titane qui présente une conductivité thermique d'environ 20 W·m⁻¹·K⁻¹, de l'acier inoxydable comprenant par exemple 18% de chrome et 8% de nickel qui présente une conductivité thermique d'environ 26 W·m⁻¹·K⁻¹, de l'acier doux qui présente une conductivité thermique d'environ 46 W·m⁻¹·K⁻¹, du platine qui présente une conductivité thermique d'environ 72 W·m⁻¹·K⁻¹, un alliage Al-SiC qui présente une conductivité thermique comprise entre environ 150 et 200 W·m⁻¹·K⁻¹, de l'aluminium qui présente une conductivité thermique d'environ 240 W·m⁻¹·K⁻¹, du cuivre qui présente une conductivité thermique d'environ 400 W·m⁻¹·K⁻¹, de l'argent qui présente une conductivité thermique d'environ 430 W·m⁻¹·K⁻¹.

La face externe 601 de la partie de recouvrement forme de préférence un élément de massage. Cet élément de massage peut occuper la totalité de la surface de ladite partie de recouvrement ou seulement une partie. La face externe 601 présente de préférence au moins un relief. Ce relief peut par exemple comprendre une surface convexe. Une telle surface convexe permet de restreindre la surface de contact avec la surface corporelle à masser et d'accroître l'effet massant. L'agencement du dispositif garantit une gestuelle efficace pour le massage de la surface corporelle désirée.

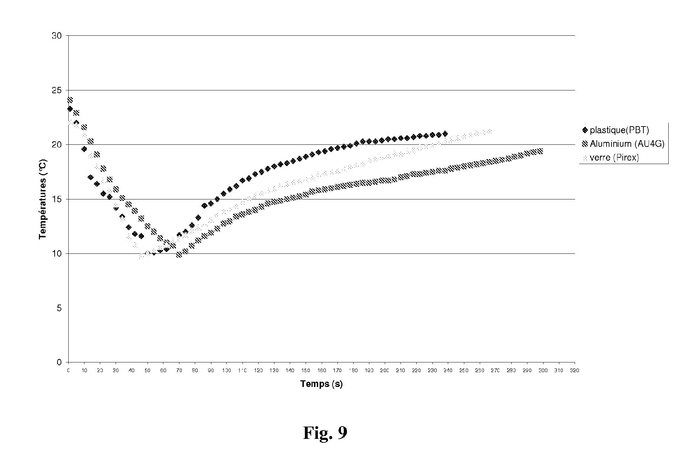

La figure 9 est un graphique représentant la température de la face externe de la partie de recouvrement en fonction du temps. Dans le présent exemple, une valve doseuse libérant 150µl de produit réfrigérant à chaque actionnement, en l'occurrence du butane-3, 2, est mise en oeuvre pour refroidir la face interne de la partie de recouvrement à tester.

Ce test est réalisé sur une partie de recouvrement réalisée soit en polybutylène téréphtalate (PBT), qui présente une conductivité thermique de 0,21 W·m⁻¹·K⁻¹, soit en pyrex, qui présente une conductivité thermique de 1,16 W·m⁻¹·K⁻¹, soit en aluminium AU4G 2017A, qui présente une conductivité thermique de 134 W·m⁻¹·K⁻¹. Les matériaux utilisés se présentent sous forme de couche présentant chacune une épaisseur de 1 mm.

Dans ce test, la face interne des différentes parties de recouvrement est tout d'abord refroidie. Ce refroidissement est obtenu par libération de produit réfrigérant directement au contact de la face interne des parties de recouvrement. Un tel refroidissement est réalisé jusqu'à ce que la température de la face externe desdites parties de recouvrement atteint 10°C. Une fois cette température atteinte, ces différentes parties de recouvrement sont laissées à température ambiante et le réchauffement de leur face externe est observé.

Sur ce graphique, on peut observer un réchauffement plus rapide des parties de recouvrement réalisées en PBT que pour les parties de recouvrement réalisées en pyrex et en aluminium. Ainsi, l'utilisation de matériaux présentant une conductivité thermique supérieure ou égale à 1 permet de maintenir un effet froid plus long au niveau de la face externe de la partie de recouvrement. Ces matériaux permettent ainsi d'obtenir un effet froid plus long pour une quantité identique ou inférieure de produit réfrigérant distribué.

Le couvercle 6 peut comporter des moyens de fixation aptes à solidariser ledit couvercle avec ledit organe d'actionnement 4. Ce couvercle 6 est de préférence amovible afin de permettre un nettoyage de l'organe d'actionnement et de la face interne de la partie de recouvrement. Toutefois, il serait possible de prévoir un couvercle et une coque d'habillage réalisée monobloc (non représenté). Dans ce dernier cas le couvercle et la coque d'habillage pourraient être réalisés en un matériau présentant une conductivité thermique supérieure ou égale à 1 W·m⁻¹·K⁻¹.

Les moyens de fixations peuvent être prévus sur un anneau 42 s'étendant à partir de la face interne 602 de la partie de recouvrement. Cet anneau peut comprendre une surface interne pourvue de filets 620 aptes à coopérer de façon complémentaire avec lesdits filets 420 de la jupe périphérique de l'organe d'actionnement. Dans ce cas, ledit couvercle est fixé sur ledit organe d'actionnement par vissage. Il serait également possible de prévoir une fixation du couvercle sur l'organe d'actionnement par emmanchement ou par encliquetage. Dans ce deuxième cas, un jonc annulaire peut par exemple être prévu sur la surface interne de l'anneau 62 qui coopère avec une rainure correspondante ménagée sur la surface externe de la jupe périphérique 42 ou inversement.

De tels moyens de fixation 420, 620 peuvent assurer une immobilisation du couvercle 6 relativement au conduit de distribution. Ainsi, en utilisation, ledit couvercle peut rester stationnaire par rapport au reste du dispositif lorsqu'il est déplacé sur la surface corporelle à traiter.

La face interne 602 de la partie de recouvrement et le conduit de distribution 40 peuvent s'étendre plus ou moins à distance l'un de l'autre. Le plateau 41 et la partie de recouvrement 60 peuvent ainsi définir entre eux un espace de réception du produit réfrigérant de volume variable 63. Les moyens de fixation peuvent assurer un tel réglage.

En effet, un vissage plus ou moins important du couvercle 6 sur l'organe d'actionnement 4 peut permettre de délimiter un espace 63 de volume plus ou moins important entre la partie de recouvrement 60 et le plateau 40. Lorsqu'un encliquetage est prévu, une pluralité de joncs annulaires peut être prévue sur la surface interne de l'anneau 62. De tels joncs forment alors des crans aptes à s'encliqueter dans une ou plusieurs rainure(s) correspondante(s) ménagée(s) sur la surface externe de la jupe périphérique 42. Lorsqu'un emmanchement est prévu, les frottements entre ladite surface interne de l'anneau 62 et ladite surface externe de la jupe périphérique 42 peuvent également permettre un réglage du volume délimité entre ladite partie de recouvrement 60 et ledit plateau 40.

En position de montage du couvercle, lesdites faces externe 601 et interne 602 de la partie de recouvrement 60 sont orientées de façon invariable relativement à au conduit de distribution 40. Ainsi, le couvercle ne peut être déplacé en rotation sur lui-même, le produit réfrigérant sortant du conduit de distribution 40 ne pouvant ainsi venir qu'au contact de la face interne de la partie de recouvrement.

Dans cet exemple, la jupe latérale 61 du couvercle s'étend à partir d'un bord externe de ladite partie de recouvrement 60. Cette jupe latérale comporte une extrémité libre 610. Cette jupe peut être disposée autour dudit organe d'actionnement 4 et en particulier autour de ladite bague de montage. La bague de montage et la jupe latérale peuvent ainsi être concentriques. Cette bague peut notamment venir en prise non serrante autour de cette jupe de sorte que la bague forme une paroi de guidage pour ladite jupe lorsque le couvercle est déplacé. Le bord libre 50 de la coque d'habillage peut former une butée pour ladite extrémité libre de ladite jupe latérale de manière à limiter la course de déplacement du couvercle.

Le fonctionnement du dispositif selon la présente invention va maintenant être décrit en référence aux figures 1 et 2.

L'utilisateur peut tout d'abord enduire une surface corporelle, tel que son visage, d'un produit cosmétique, tel qu'un produit anti-rides. Ensuite, l'utilisateur peut se saisir du dispositif en le tenant au niveau de la coque d'habillage 5. Afin d'obtenir un effet de froid, l'utilisateur exerce une pression sur le couvercle 6, et notamment sur la partie de recouvrement 60, afin d'actionner la valve de distribution 2. Le produit réfrigérant sortant du récipient aérosol peut alors emprunter le conduit d'expulsion de ladite valve puis le conduit de distribution 40. Le produit réfrigérant débouche alors dans l'espace 63 délimité par l'organe d'actionnement 4 et le couvercle 6. Le produit réfrigérant vient ainsi au contact de la face interne 602 de la partie de recouvrement. Lorsque le produit réfrigérant vient frapper la face interne 602, ce dernier est redirigé latéralement, permettant ainsi d'avoir une bonne répartition du froid sur la partie de recouvrement. Le produit réfrigérant peut ensuite être évacué de l'espace 63 par un passage d'éventation situé entre la jupe périphérique 42 et l'anneau 62.

Le matériau utilisé pour la réalisation de ce couvercle permet une bonne conduction de froid à travers cette partie de recouvrement de sorte que la face externe de la partie de recouvrement se refroidit instantanément. L'utilisateur peut alors positionner la face externe de la partie de recouvrement contre la surface corporelle désirée. La face externe refroidit simultanément le produit cosmétique et la surface corporelle enduite de ce produit cosmétique.

Ce froid apporte à l'utilisateur une sensation de bien-être et d'autre part peut favoriser la pénétration du produit cosmétique au sein de la peau d'où une action plus efficace de ce produit. Par ailleurs, la convexité de ladite face externe assure alors un massage localisé et efficace de la surface corporelle et peut ainsi améliorer la diffusion du produit cosmétique au sein de la peau.

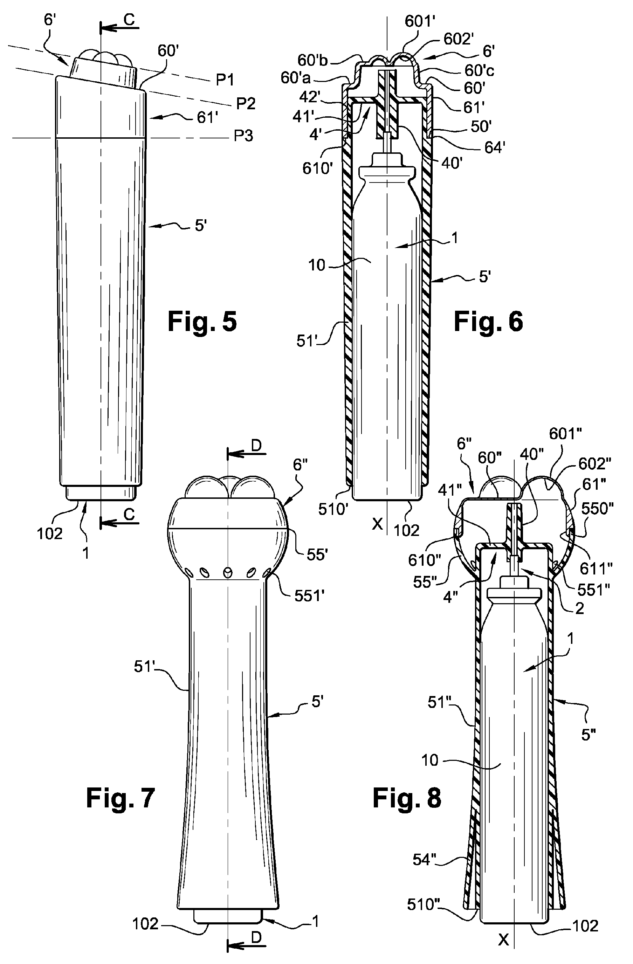

Les figures 3 et 8 représentent trois autres modes de réalisation d'un dispositif selon la présente invention. Par soucis de clarté, des références numériques similaires ont été attribuées à des éléments similaires aux différents modes de réalisation, les éléments divergeant entre ces modes de réalisation ayant des références numériques affectées d'un signe prime ou seconde. Seules ces différences seront évoquées dans la suite de la description.

Les figures 3 et 4 montrent un deuxième mode de réalisation du dispositif selon la présente invention. Ce dispositif comprend une coque d'habillage 5' comportant une paroi cylindrique 51' pourvue d'une extrémité libre ouverte 510'. Cette coque d'habillage est ainsi dépourvue de fond. A l'opposé, ladite coque d'habillage comporte un bord 50'.

Dans ce mode de réalisation, la coque d'habillage 5' et l'organe d'actionnement 4' sont réalisés monobloc. Cet organe d'actionnement comporte un conduit de distribution 40' à partir duquel s'étend radialement un plateau 41' bordé par une jupe périphérique 42'. Le conduit de distribution 40' peut s'étendre de part et d'autre de ce plateau 41 '.

Comme représenté, la jupe périphérique 42' peut présenter une épaisseur inférieure à l'épaisseur de la paroi cylindrique 51' de la coque d'habillage de sorte que le bord 50' forme un décrochement externe relativement à ladite jupe périphérique.

Dans cet exemple de réalisation, le couvercle comporte une partie de recouvrement 60' étagée. En particulier, cette partie de recouvrement comporte une embase 60'a à partir de laquelle se dresse une plateforme 60'b pourvue d'un élément de massage. Cette plateforme et cette embase peuvent être reliée par une paroi annulaire 60'c. Cette plateforme et cette embase s'étendent respectivement selon un plan P1 et P2, qui peuvent être parallèles. Seul l'élément de massage peut être réalisé en un matériau présentant une conductivité thermique supérieure ou égale à 1 W·m⁻¹·K⁻¹. Dans ce mode de réalisation, cet élément de massage présente une forme hémisphérique.

En position de montage, la jupe latérale 61' du couvercle 6' vient en prise autour de la jupe périphérique 42' de l'organe d'actionnement 4'. L'embase 60' se positionne en regard du plateau 42' et le conduit de distribution se positionne en regard de l'élément de massage. Il est possible de prévoir un espace de volume variable entre la partie de recouvrement et l'organe d'actionnement et plus particulièrement entre l'élément de massage et le conduit de distribution ainsi qu'entre l'embase et le plateau.

La jupe périphérique 42' peut comporter une extrémité libre 610' apte à venir en butée contre le décrochement externe 50' de la coque d'habillage. Toutefois, il est possible de prévoir un écartement 64' entre cette extrémité libre 610' et ce décrochement externe 50'. Dans ce cas, cet écartement peut être en communication fluidique avec l'espace défini entre la partie de recouvrement et l'organe d'actionnement. Dans ce cas, cet écartement définit un orifice d'éventation.

L'extrémité libre 610' peut s'étendre selon un plan P3 transversal à l'axe de cylindre X. Ce plan P3 peut être sécant avec les plans P1 et P2.

Dans ce mode de réalisation, le récipient aérosol 1 fait saillie relativement à l'extrémité libre 510' de la coque d'habillage de sorte que pour actionner la valve de distribution 2, l'utilisateur doit appuyer sur la deuxième extrémité fermée 102 du récipient aérosol 1. Un tel appui déplace ainsi le récipient par rapport à la valve 2 retenue dans le conduit de distribution 40' et entraîne la distribution de produit réfrigérant contre la face interne de la partie de recouvrement 602'. Le froid se propage alors de la face interne vers la face externe. L'utilisateur peut alors masser une surface corporelle éventuellement préalablement enduite d'un produit cosmétique.

Les figures 5 et 6 montrent un troisième mode de réalisation du dispositif selon la présente invention. Ce dispositif se distingue du dispositif représenté sur les figures 3 et 4 en ce que ladite plateforme 60' comporte plusieurs éléments de massage. Sur la figure, ces éléments de massage sont au nombre de trois.

Les figures 7 et 8 montrent un quatrième mode de réalisation du dispositif selon la présente invention. Ce dispositif se distingue du dispositif représenté sur les figures 5 et 6 en ce que la coque d'habillage 5" comprend une paroi cylindrique 51 " à partir de laquelle s'étendent des ailettes 54" s'évasant vers l'extérieur. Ces ailettes peuvent s'étendre jusqu'à l'extrémité libre 510" de la coque d'habillage. De telles ailettes favorisent la prise en main du dispositif.

Par ailleurs, la coque d'habillage 5" comporte une partie évasée sous forme de corolle 55". Cette corolle comprend un bord libre 550".

Ce dispositif comporte un couvercle 6" comprenant une partie de recouvrement 60" bordé par une jupe latérale 61 " pourvu d'une extrémité libre 610". Dans cet exemple, ladite partie de recouvrement 60" comporte plusieurs éléments de massage, par exemple au nombre de trois. La jupe latérale 61 " présente quant elle une face externe présentant un profil arrondi. En position de montage, l'extrémité libre 610" de la jupe latérale vient en butée contre le bord libre 550' de la corolle. Le bord libre 610' peut être pourvu d'une lèvre annulaire interne 611 " apte à venir en prise serrante contre une surface interne de ladite corolle. Cette lèvre guide ainsi le couvercle lors de son montage sur la corolle et fixe le couvercle sur cette corolle.

Il est à noter qu'un ou plusieurs orifices d'éventation 551', 551" peuvent être prévus. Avantageusement, ces orifices sont prévus à distance de la surface destinée à venir en contact de la peau de sorte que le produit réfrigérant ne sera pas expulsé directement sur la peau. Ces orifices sont de préférence orientés en éloignement de la partie de recouvrement destinée à venir au contact de la peau. De tels orifices peuvent par exemple être ménagés au travers de la corolle 55".

Ces différents modes de réalisation fonctionnent de façon similaire au premier mode de réalisation et ne seront donc pas davantage expliqués.

Dans toute la description, l'expression « comportant un » doit être considérée comme étant synonyme de « comportant au moins un », sauf si le contraire est spécifié.

## Revendications

1. - Dispositif de refroidissement comprenant :
- des moyens de refroidissement (1) comportant un distributeur de produit réfrigérant comprenant un récipient aérosol (10) contenant ledit produit réfrigérant et une valve de distribution (2) associée à une coupelle de fixation à sertir sur le récipient aérosol, ledit récipient aérosol étant au moins en partie logé à l'intérieur d'une coque d'habillage (5 ; 5' ; 5"),
- un conduit de distribution (40 ; 40' ; 40") engagé sur ladite valve de distribution (2),
- un couvercle (6 ; 6' ; 6") surmontant ledit conduit de distribution (40 ; 40' ; 40"),
**caractérisé en ce que** ledit couvercle (6 ; 6' ; 6") comporte une face interne (602 ; 602' ; 602 ") imperméable au produit réfrigérant et **en ce qu'**au moins une partie du couvercle, notamment une face externe du couvercle (601 ; 601' ; 601 "), est réalisée en un matériau présentant une conductivité thermique supérieure ou égale à 1 W·m⁻¹·K⁻¹.

2. - Dispositif selon la revendication 1, dans lequel ladite conductivité thermique est comprise entre 10 et 430 W·m⁻¹·K⁻¹, et plus préférablement entre 20 et 240 W·m⁻¹·K⁻¹.

3. - Dispositif selon la revendication 1 ou 2, dans lequel ledit matériau présentant une conductivité thermique supérieure ou égale à 1 W·m⁻¹·K⁻¹ est un matériau métallique et notamment du titane, de l'acier, du platine ou de l'aluminium.

4. - Dispositif selon la revendication 1, 2 ou 3, dans lequel ledit récipient comporte une première extrémité (100) pourvue de ladite valve de distribution et une deuxième extrémité fermée (102), lesdites première et deuxième extrémités étant reliées par une paroi latérale (101), ladite coque d'habillage (5 ; 5' ; 5") enveloppant au moins partiellement ladite paroi latérale.

5. - Dispositif selon l'une quelconque des revendications précédentes, dans lequel ledit couvercle (6 ; 6' ; 6") est amovible.

6. - Dispositif selon l'une quelconque des revendications précédentes, dans lequel ledit couvercle (6 ; 6' ; 6") est fixé sur ledit conduit de distribution par emmanchement en force, par vissage ou par encliquetage.

7. - Dispositif selon l'une quelconque des revendications précédentes, dans lequel en position de montage du couvercle (6 ; 6' ; 6"), ledit couvercle est apte à venir en butée contre ladite coque d'habillage (5 ; 5' ; 5").

8. - Dispositif selon l'une quelconque des revendications 1 à 4, dans lequel ledit couvercle et ladite coque d'habillage sont réalisés monobloc.

9. - Dispositif selon l'une quelconque des revendications précédentes, dans lequel lesdites faces interne et externe du couvercle (6 ; 6' ; 6") sont orientées de façon invariable relativement audit conduit de distribution.

10. - Dispositif selon l'une quelconque des revendications précédentes, comprenant des moyens d'immobilisation (420, 620) dudit couvercle sur ledit conduit de distribution et/ou sur ladite coque d'habillage.

11. - Dispositif selon l'une quelconque des revendications précédentes, dans lequel ladite coque d'habillage (5' ; 5") et ledit conduit de distribution (40' ; 40") sont réalisées monobloc.

12. - Dispositif selon l'une quelconque des revendications précédentes, comprenant des moyens de réglage (420 ; 620) du volume délimité entre ledit conduit de distribution et ledit couvercle.

13. - Dispositif selon l'une quelconque des revendications précédentes, dans lequel ladite face externe (601 ; 601' ; 601 ") présente une surface convexe.

14. - Procédé d'application d'un produit cosmétique sur une surface corporelle comprenant une première étape d'appliquer ledit produit cosmétique sur ladite surface corporelle suivie d'une deuxième étape de masser ladite surface corporelle recouverte de produit cosmétique à l'aide d'un dispositif selon l'une quelconque des revendications précédentes.
